Europäisches Patentamt

European Patent Office

Office européen des brevets

(11) Veröffentlichungsnummer: **0 339 330**
**A2**

## (12) EUROPÄISCHE PATENTANMELDUNG

(21) Anmeldenummer: 89106117.8

(22) Anmeldetag: 07.04.89

(51) Int. Cl.⁴: **C07C 69/14 , C07C 67/08**

(30) Priorität: 26.04.88 DE 3814095

(43) Veröffentlichungstag der Anmeldung:
02.11.89 Patentblatt 89/44

(84) Benannte Vertragsstaaten:
**AT BE CH DE ES FR IT LI NL**

(71) Anmelder: **Spradau, Hans F. W.**
**Hauptstrasse 5**
**D-2832 Twistringen-Heiligenloh(DE)**

(72) Erfinder: **Spradau, Hans F. W.**
**Hauptstrasse 5**
**D-2832 Twistringen-Heiligenloh(DE)**

(74) Vertreter: **Bolte, Erich, Dipl.-Ing. et al**
**c/o Meissner, Bolte & Partner Patentanwälte**
**Hollerallee 73**
**D-2800 Bremen 1(DE)**

(54) Verfahren zur Herstellung von Ethylacetat.

(57) Die Erfindung betrifft ein Verfahren zur Herstellung von Ethylacetat wobei Essigsäure mit Äthanol unter katalytischer Wirkung von Citronensäure umgesetzt werden.

EP 0 339 330 A2

## Verfahren zur Herstellung von Ethylacetat

Die vorliegende Erfindung betrifft ein Verfahren zur Herstellung von Ethylacetat.

Ethylacetat stellt ein wichtiges Lösungsmittel in vielen Industriezweigen wie z. B. in der Film-, Lack-, Kunstleder- und Kunstseide-Fabrikation, in der Kosmetikherstellung sowie in der Lebensmittelindustrie dar. Bekannte Herstellungsverfahren beruhen auf der Umsetzung von Äthanol und Essigsäure unter Katalytischer Wirkung von starken Mineralsäuren, meist Schwefelsäure (vgl. Verfahren nach DE-PS 824341). Durch Änderung der Konzentration Rader Reaktionspartner, durch Temperaturerhöhung und/oder Entfernung der Endprodukte kann die Produktaus beute gesteuert werden.

Verfahrenstechnisch vorteilhaft erwiesen sich kontinuierliche Verfahren zur Herstellung von Ethylacetat.

Die Verwendung von Mineralsäuren wird vielfach als Nachteil der bekannten Verfahren angesehen. Sie gelangen im Herstellungsverfahren zumindest in geringen Mengen in das hergestellte Ethylacetat. Insbesondere bei Verwendung von Ethylacetat in der Lebensmittelindustrie sind selbst Spuren von Mineralsäuren unerwünscht, da die Säuren selbst toxisch sein können bzw. geschmacklich oder toxikologisch bedenkliche Reaktionen bewirken können.

Der vorliegenden Erfindung liegt nunmehr die Aufgabe zugrunde, ein Verfahren zur Herstellung von Ethylacetat bereitzustellen, das ohne Mineralsäuren arbeitet und damit die genannten Nachteile ausschließt.

Die Lösung dieser Aufgabe wird in überraschend einfacher Weise durch die Verwendung von Citronensäure erreicht. Citronensäure ist eine natürliche Säure und spielt im Stoffwechsel des Menschen eine bedeutende Rolle. Sie wird aus natürlichem Material gewonnen und ist toxikologisch unbedenklich.

Aus toxikologischer Sicht ist die Herstellung von Ethylacetat gemäß den Ansprüchen 3 und 4 unter Verwendung von biologisch hergestelltem Äthanol und/oder biologisch hergestellter Essigsäure besonders vorteilhaft.

Unter biologisch hergestelltem Äthanol ist durch alkoholische Gärung von glukosehaltigen Substraten wie Treber, Melasse, Rohrzucker, Zuckerrübenschnitzeln, Obst oder Maischen von stärkehaltigen Produkten wie Kartoffeln, Reis oder Mais gewonnenes Äthanol zu verstehen.

Biologische Essigsäure wird durch aerobe Essigsäuregärung alkoholischer Flüssigkeiten unter enzymatischem Einfluß von Essigbakterien (Acetobacterin) gewonnen.

Das erfindungsgemäße Verfahren arbeitet vorzugsweise mit einem 1,5 molaren Äthanol-Überschuß. Die Menge an Citronensäure liegt bei 5 bis 10 Gew.% bezogen auf die Essigsäuremenge.

Im folgenden wird die Erfindung beispielhaft, insbesondere hinsichtlich der verfahrenstechnischen Ausführung, anhand einer Zeichnung näher erläutert:

Ein Vorratsbehälter I beinhaltet eine Mischung aus Essigsäure und Citronensäure. Über eine Leitung 1 gelangt die Säuremischung in die untere Hälfte eines mit Füllkörpern beispielsweise Raschigringen gefüllten zylinderförmigen Reaktors II und wird dort z. B. mittels einer Düse versprüht. Aus einem Vorratsbehälter III wird Äthanol (94 %ig) über eine Leitung 3 in den Reaktor II transportiert. Der Einlaß für die Leitung 3 im Reaktor II ist unter dem Einlaß für Leitung 1 angeordnet. Diese Anordnung der Leitungen hat sich als besonders vorteilhaft erwiesen. Das in den unteren Teil geleitete Äthanol verdampft beim Erhitzen sehr schnell. Dadurch kommt es zu einer guten Vermischung mit dem im mittleren Bereich des Reaktors II versprühten Citronensäure-Essigsäure-Gemischs. Der Reaktor II ist im unteren Teil mit einer äußeren Heizung versehen, die den Inhalt des Reaktors auf 78 °C erwärmt. Im Reaktor findet die Veresterungsreaktion statt. Gleichzeitig bildet sich ein termeres aceotropes Gemisch aus Ethylacetat, Äthanol und Wasser, das durch Leitung 2 in einen Kondensator IV strömt, und dort kondensiert. Aus dem Kondensator IV tritt durch eine Leitung 4 ein Gemisch aus 83,2 % Ethylacetat, 7,8 % Wasser und 9 % Äthanol und wird in einen Extraktor VI geführt. Dort wird das Gemisch im Gegenstrom mit Wasser gewaschen; das sich aufgrund des geringeren spezifischen Gewichts im oberen Bereich des Extraktors abscheidende Ethylacetat wird durch eine Leitung 6 entnommen; dieses Ethylacetat ist 96 %ig und stellt das gewünschte Endprodukt dar. Vom Boden des Extraktors VI führt eine Leitung 6a in eine Aceotropkolonne VII. Dort wird das in der Leitung 6a transportierte Wasser-Äthanol-Ethylacetat-Gemisch teilweise getrennt. Ethylacetat verdampft, wird in einen Kondensator VIII transportiert und von dort über Leitung 8 in den Extraktor 6 geleitet oder über Leitung 8a zurück in die Kolonne VII. Aus der Aceotropkolonne VII fließt durch Leitung 7 ein Äthanol-Wasser-Gemisch in eine Kolonne IX, in der die Trennung dieses Gemisches durchgeführt wird. Das anfallende Wasser wird am Boden der Kolonne IX abgelassen. Das verdampfende Äthanol wird mittels eines Kondensators X kondensiert und über Leitung 10 in das Vorratsgefäß III transportiert. Es läßt sich gegebenenfalls mittels Leitung 10a in die Kolonne IX zurückleiten.

In einer Kolonne XI erfolgt die Konzentrierung eines Citronensäure-Essigsäure-Wasser-Gemisches, das im Reaktor II bei Durchführung der eigentlichen Umsetzung anfällt. Eine Leitung 2a führt dieses Gemisch vom Boden des Extraktors II in die Kolonne XI. Das verdampfende Wasser wird in einer Kolonne XII kondensiert und dann abgeleitet. Das konzentrierte Essigsäure-Citronensäure-Gemisch wird durch Leitung 11 in den Säure-Vorratsbehälter I geführt.

Die Kolonnen 7, 9 und 11 mit den Kondensatoren 8, 10 und 12 dienen hauptsächlich der Rückgewinnung von Äthanol und Säure-Gemisch. Die Aggregate 1, 2, 4, 5 und 6 betreffen hingegen die unmittelbare Herstellung von Ethylacetat.

Bei Durchführung des erfindungsgemäßen Verfahrens mit 6,15 kg Essigsäure, 0,4 kg Citronensäure und 5,7 kg Äthanol kann in einem Produktionstakt von 8 Stunden Ethylacetat in 96 %-iger Konzentration hergestellt werden. Das in den Extraktor VI eingeleitete Gemisch besteht in diesem Fall aus 7,47 kg Ethylacetat, 0,81 kg Äthanol und 0,72 kg Wasser. Im Extraktor wird das Gemisch mit Wasser gewaschen; Ethylacetat scheidet sich oben ab und wird entnommen und zwar in einer Konzentration von 96 %. Das am Boden des Extraktors abgeführte Gemisch enthält 0,18 kg Äthanol sowie 0,17 kg Ethylacetat.

Die Ausbeute an Ethylacetat beim beschriebenen Verfahren beträgt 92 %.

Das erfindungsgemäße Verfahren kann kontinuierlich wie auch in Chargen ausgeführt werden. Die verwendete Essigsäure hat eine Konzentration von über 10 %. Für die Praxis ist eine 30 %-ige Essigsäure geeignet. Bei höherer Konzentration ergibt sich ein entsprechend größerer Aufwand bei der Herstellung der Essigsäure.

Der Vorgang der Veresterung - bei dem dargestellten Ausführungsbeispiel im Reaktor II - kann dadurch verbessert, nämlich beschleunigt und in bezug auf die Ausbeute verbessert werden, wenn während der Reaktion Wasser entzogen wird. Der Reaktor II ist bei dem gezeigten Ausführungsbeispiel zweckmäßigerweise so eingerichtet, daß fortlaufend Wasser während der Reaktion entzogen wird. Hierfür kann das Verfahren der Pervaporation angewendet werden.

**Bezugszeichenliste:**

I Vorratsbehälter für Säure
II Reaktor
III Vorratsbehälter für Äthanol
IV Kondensator
V Ventil
VI Extraktor

VII Azeotropkolonne
VIII Kondensator
IX Alkoholkolonne
X Kondensator
XI Kolonne zur Säure-Konzentrierung
XII Kondensator

1 Leitung von I nach II
2 Leitung von II nach IV
2a Leitung von II nach XI
3 Leitung von III nach II
4 Leitung von IV nach VI
6 Leitung von VI zur Abfüllung bzw. nach II
6a Leitung von VI nach VII
7 Leitung von VII nach IX
8 Leitung von VIII nach VI
8a Leitung von VIII nach VII
9 Leitung von IX nach X
10 Leitung von X nach III
10a Leitung von X nach IX
11 Leitung von XI nach I

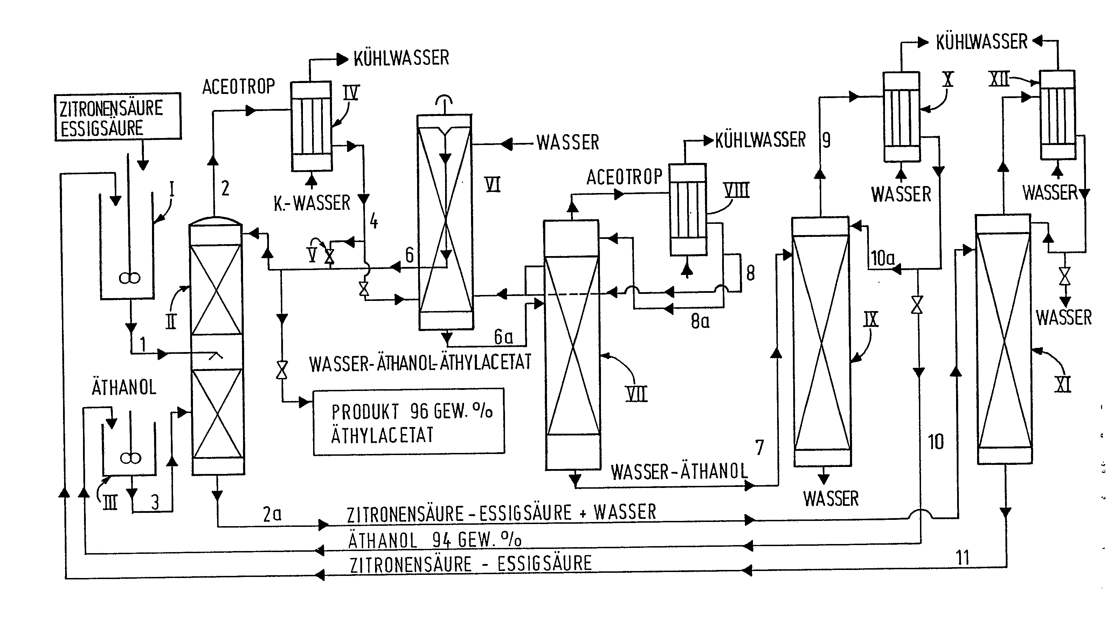

**Ansprüche**

1. Verfahren zur Herstellung von Ethylacetat aus Essigsäure und Äthanol, **dadurch gekennzeichnet,** daß es unter Zusatz katalytischer Mengen Citronensäure durchgeführt wird.

2. Verfahren nach Anspruch 1, dadurch gekennzeichnet, daß Citronensäure in einer Konzentration von 5-10 Gew.%, bezogen auf die Essigsäuremenge, verwendet wird.

3. Verfahren nach Anspruch 1 oder 2, dadurch gekennzeichnet, daß die molare Konzentration an Äthanol etwa das 1,5-fache der molaren Essigsäurekonzentration beträgt.

4. Verfahren nach einem oder mehreren der vorangehenden Ansprüche, dadurch gekennzeichnet, daß Äthanol, welches aus biologischen Gärprozessen hergestellt wurde, verwendet wird.

5. Verfahren nach einem oder mehreren der vorangehenden Ansprüche, dadurch gekennzeichnet, daß Essigsäure, die in biologischen Gärprozessen hergestellt wurden, verwendet wird.

6. Verfahren nach Anspruch 1 sowie einem oder mehreren der weiteren Ansprüche, dadurch gekennzeichnet, daß während der Reaktion von Essigsäure und Äthanol mit Zitronensäure als Katalysator Wasser entzogen wird, vorzugsweise fortlaufend, insbesondere durch Pervaporation.

ZITRONENSÄURE
ESSIGSÄURE

KÜHLWASSER

ACEOTROP

IV

K.-WASSER

WASSER

VI

KÜHLWASSER

ACEOTROP

VIII

KÜHLWASSER

KÜHLWASSER

XII

WASSER

X

WASSER

9

10a

8

8a

II

6

6a

ÄTHANOL

V

WASSER-ÄTHANOL-ÄTHYLACETAT

VII

IX

XI

1

PRODUKT 96 GEW. %
ÄTHYLACETAT

WASSER-ÄTHANOL

7

WASSER

WASSER

III

3

2a

ZITRONENSÄURE - ESSIGSÄURE + WASSER

10

ÄTHANOL 94 GEW. %

11

ZITRONENSÄURE - ESSIGSÄURE

EP 0 339 330 A2